Europäisches Patentamt
European Patent Office
Office européen des brevets

(11) Publication number: 0 274 264
A1

# EUROPEAN PATENT APPLICATION

(21) Application number: 87311262.7

(22) Date of filing: 21.12.87

(51) Int. Cl.4: G01N 33/577 , G01N 33/574 , G01N 33/543 , G01N 33/555 , C12P 21/00 , //(C12P21/00, C12R1:91)

(30) Priority: 24.12.86 JP 306574/86

(43) Date of publication of application: 13.07.88 Bulletin 88/28

(84) Designated Contracting States: CH DE FR GB IT LI NL

(71) Applicant: FUJIREBIO KABUSHIKI KAISHA also trading as FUJIREBIO INC.
6-7, Shimoochiai 4-chome Shinjuku-ku
Tokyo 161(JP)

(72) Inventor: Uchida, Yoshiaki
Fujirebio K.K. 6-7, Shimoochiai 4-chome
Shinjuku-ku Tokyo(JP)
Inventor: Honda, Hideo
Fujirebio K.K. 6-7, Shimoochiai 4-chome
Shinjuku-ku Tokyo(JP)
Inventor: Kikuchi, Yousuke
Fujirebio K.K. 6-7, Shimoochiai 4-chome
Shinjuku-ku Tokyo(JP)
Inventor: Kakutani, Masami
Fujirebio K.K. 6-7, Shimoochiai 4-chome
Shinjuku-ku Tokyo(JP)

(74) Representative: Silverman, Warren et al
HASELTINE LAKE & CO. Hazlitt House 28
Southampton Buildings Chancery Lane
London WC2A 1AT(GB)

## (54) Reverse passive particle agglutination reagent.

(57) A reagent for detecting CA19-9 antigen by reverse particle agglutination comprises, sensitised on carrier particles, a novel IgM class monoclonal antibody which recognises an antigenic determinant of CA19-9. The reagent is useful for the diagnosis of digestive organ cancers, particularly pancreas cancer.

EP 0 274 264 A1

# REVERSE PASSIVE PARTICLE AGGLUTINATION REAGENT

This invention relates to a reagent for use in reverse passive particle agglutination carried out for detecting a sugar chain antigen CA19-9 which is important in the diagnosis of cancer of digestive organs, particularly cancer of the pancreas.

Enzyme immunoassay (EIA) and radioimmunoassay (RIA have hitherto been used in detecting CA19-9. However, these methods are troublesome to carry out, and it takes a long time to obtain the required measurements. Moreover, specialised apparatus is necessary in these methods. Thus the use of these methods as screening methods for measuring many samples in a short time is too expensive to be practicable.

In order to develop a measuring method for CA19-9 which does not have such problems, the present inventors prepared an IgG class anti CA19-9 monoclonal antibody (TM19-9) having an affinity and a specificity comparable with those of NS19-9 antibody which is already known as an IgG class anti CA19-9 monoclonal antibody. For this purpose, the inventors followed the procedure of Japanese KOHYO No.501,519/1984. The inventors have investigated the reverse passive particle agglutination (RPPA) method of detecting CA19-9 antigen using this monoclonal antibody. However, the sensitivity of the method was insufficient, and accordingly, this antibody was not found to be suitable for use in a reagent for detecting CA19-9 antigen by reverse passive particle agglutination.

On the other hand, it is known in connection with passive particle agglutination that an IgM antibody has been used for the identification of blood groups (Matsuhashi et al, "Men-eki Kessei Gaku (Immunoserology)-", p 60, Kodan-Sha Scientific (Japan), 1979). However, there has not been any reported proposal to use an IgM class monoclonal antibody for any form of reverse passive particle agglutination.

It is an object of the invention to provide a reagent for use in the reverse passive particle agglutination test for detecting CA19-9 antigen which reagent has a sensitivity appropriate to its use as a screening reagent.

According to the present invention, there is provided an IgM monoclonal antibody having high affinity for an antigenic determinant of CA19-9 when used in a reverse passive particle agglutination reagent. This monoclonal antibody is not only able to recognise CA19-9 antigen, it is suitable for use in a reverse passive particle agglutination reagent to detect CA19-9 antigen having sufficient sensitivity for use as a screening agent.

IgM class monoclonal antibodies suitable for passive particle agglutination are not always suitable for reverse passive particle agglutination. Thus the present inventors have previously prepared IgM class monoclonal antibody for detecting HBs antigen having a high affinity comparable with 5D3, known as the IgM class monoclonal antibody which is capable of detecting HBs antigen. For this purpose they followed the teaching of EP-A-0059754. The performance of this monoclonal antibody in passive particle agglutination was compared with its performance in reverse passive particle agglutination. In the case of passive particle agglutination, the erythrocytes sensitised with HBs antigen easily agglutinate in the presence of the antibody, and this antibody, when present in an amount of 1/10 to 1/100 of an IgG class monoclonal antibody, exhibited almost the same agglutination titer as the IgG class monoclonal antibody. However, in the case of reverse passive particle agglutination, the agglutination titer of this IgM class monoclonal antibody was lower than that of a polyclonal antibody and a quite opposite agglutination titer relationship existed with respect to the IgG class monoclonal antibody.

Thus, the antibody of the present invention is suitable for use in a reagent for the reverse particle agglutination for detecting CA19-9, which reagent will generally form part of a kit of materials for use in the diagnosis of cancer, in particular a cancer of the digestive tract, the kit additionally comprising a serum diluent, a control serum, unsensitised carrier particles and a non-specific reaction absorber.

In the description of this invention which follows, reference will be made to the accompanying drawings, wherein:

FIGURE 1 indicates the ability of the IgM class monoclonal antibody to inhibit the reaction of enzyme-labelled monoclonal antibody with a gastric cancer cell antigen.

FIGURE 2 indicates the correlation of the analytical results obtained in an enzyme immunoassay using the IgM class monoclonal antibody of the invention with the results obtained by using a commercial reagent kit for measuring CA19-9 antigen.

FIGURE 3 indicates the antigenic reactivities with respect to various antibodies of the fractions of glycolipid extracts of colon carcinoma cells which have been treated (or not treated) with neuraminidase separated by as DEAE column.

FIGURE 4 indicates the correlation of analytical results obtained by the reverse passive particle agglutination using the IgM class monoclonal antibody of the invention with results obtained by radioimmunoassay.

The carrier particles used as insoluble solid phase to be sensitised with the monoclonal antibodies of this invention may be conventional carrier particles for use in reverse passive particle agglutination, and include animal erythrocytes such as human, sheep and chicken erythrocytes, microbial cells such as those of Serratia marcescens, gelatin particles (US-A-4,416,813), polystyrene latex, kaolin and carbon powder.

The sensitisation method to which the antibodies or carrier particles are subjected may also be of usual type. Thus, it can be carried out by the method using a coupling agent such as tannic acid, glutaraldehyde, bisdiazobenzidine, toluene diisocyanate, difluorodinitrobenzene, carbodiimides, quinones or chromium chloride, or by a physical adsorption method.

The antibody sensitised particles are usually lyophilised by a conventional method, and combined with a reconstitution solution, a serum diluent, a control serum, unsensitised carrier particles and a nonspecific reaction absorber to form a reagent kit.

When using the reagent of the invention for measuring CA19-9 antigen, procedure may be according to conventional reverse particle agglutination methods. For example, a sample serum is placed in a well of a microplate, and a $2^n$ dilution series is prepared. The antibody sensitised particles are added to each well, and stirred. Then, the mixture is allowed to stand, usually for 1 to 3 hours, and the agglutination pattern of the sensitised particles is observed. In another method, a sample serum is mixed with the sensitised particles on a slide glass, and the agglutination pattern after 1 to 3 minutes is observed.

The reagent of the invention is useful for the diagnosis of cancers of digestive organs, particularly cancer of the pancreas, since it can be used to measure CA19-9 antigen accurately and with high sensitivity. In the case of CA19-9, the cut-off value is usually set at 37 U/ml. In the range of 37 to 100 U/mg, results obtained could be indicative of pancreatitis or hepatitis than cancer of a digestive organ. At more than 100 U/ml, the probability of a form of cancer being found is high. As the diagnostic agent for these diseases, the reagent of the invention provides a detectability comparable with that of the EIA and RIA methods for detecting cancer and moreover the procedure for determination of cancer is simple. Furthermore, no particular apparatus is necessary. Accordingly, the measurement of CA19-9 may be easily carried out at a wide variety of locations.

The following Examples illustrate this invention:

## EXAMPLE 1

### Preparation of Monoclonal Antibody and characterization of its specificity

1. Preparation of Antigen for Immunisation

KATO-III cancer cells, an established cell line derived from gastric cancer, were cultured by monolayer culture in a culture bottle, and serum proteins were removed by washing twice with serum-free RPMI-1640 medium. The cells were cultured in serum-free RPMI-1640 medium for 1 hour, and the medium was replaced by fresh serum-free RPMI-1640 medium. Cultivation was continued for a further 20 hours, and the culture supernatant was then recovered. The supernatant was concentrated by passage over polyethylene glycol 2,000, and gel filtration was carried out using a SEPHAROSE 4B column. The polymer fractions eluted near void volume were collected, and used as the immunogen. The immunogen was mixed with complete Feund's adjuvant, and injected into abdominal cavities of mice. After about 1 month, injection into a vein was carried out. The spleen of the mice were removed after 3 days, and used for the following cell fusion.

2. Cell fusion, Cloning and Purification

Mouse spleen cells thus obtained were mixed with mouse myeloma P3UI cells in the ratio of 3:1, and cell fusion was carried out in the presence of 50% polyethylene glycol having a mean molecular weight of 4,000. The fused cells were cultured in the wells of a microplate, and the activity of each supernatant culture where the growth of the cells was observed was measured by enzyme immunoassay (EIA). Then, cloning was carried out with the cells of each well where activity was recognised by limiting dilution. The clones obtained were transferred into the abdominal cavities of BALB/C mice into which pristane had been injected, and after 10 to 14 days, ascites were withdrawn. The monoclonal antibody material contained in the ascites in a high concentration was separated and purified by gel filtration and using a DEAE column.

The molecular weight of the monoclonal antibodies measured by high pressure liquid chromatography (gel filtration) was 800,000 to 1,000,000, this being consistent with the molecular weight of IgM antibody. 100 μl of antibody suspension containing 100 μg/ml of this antibody were placed in the wells of the microplate of EIA, and allowed to stand at 37°C for 2 hours. After washing the wells, each received 100 μl of the colouring agent ABTS and a peroxidase (POD)-labelled anti-mouse IgG, $IgG_{2a}$, $IgG_{2b}$, $IgG_3$, IgA or IgM, and the wells were allowed to stand at 37°C for 1 hour. Every well was washed, and the degree of colouration of the contents of each well was compared with a standard. Thus, it was confirmed that the antibody produced reacts with the anti-mouse IgM antibody alone, and that it belongs to a mouse IgM class of antibody. This antibody was named KA3A3.

3. Reactivity and Specificity

3-1. Reactivities with respect to Various Cell Lines

The reactivities with respect to various cell lines were measured by enzyme immunoassay using a 96 wells microplate. Various sonicated cell lines were used as antigens. These antigens were immobilised on each well of the microplate, and treated with 0.1 U/ml of neuraminidase. Thus, with each antigen the change of reactivity on treatment with neuraminidase was determined, indicating thereby whether each antigen was a sialic acid-containing antigen or not.

The results obtained are tabulated in Table 1. For comparison, the reactivities of the antibody NS19-9 used in a commercially available kit for detecting CA19-9 antigen ("Imunoclone CA19-9", Fujirebio Inc.) were also measured, and are described in the same table.

Table1

Comparison of Reactivities of Monoclonal Antibody KA3A3 with Monoclonal Antibody TM19-9 to Various Antigens by EIA

| | Reactivity Index | |
|---|---|---|
| Antigen | KA3A3 | NS19-9 |
| PC-9 | 0 | 0 |
| PC-10 | 0 | 0 |
| A549 | 0 | 0 |
| NRC-12 | 0 | 0 |
| KATO-III | 6 (1)* | 3 (0) |
| SW116 | 4 | 2 |
| COLO-205 | 10 (1) | 6 (1) |
| CEM | 0 | 0 |
| Raji | 0 | 0 |
| Normal Leucocyte | 0 | 0 |
| CEA | 0 | 0 |
| Meconium (CA19-9 +) | 8 (1) | 6 (0) |

* ( ): Treated with neuraminidase

As shown in the table, the KA3A3 antibody strongly reacted with KATO-III and COLO-205, and it reacted weakly with SW1116. Moreover, it also reacted strongly with the macromolecular fractions of meconium obtained by gel filtration using Sepharose 4B. However, when these reactive antigens had been treated with neuraminidase, their reactivities disappeared. Thus, the antigens recognised by the KA3A3 antibody contained sialic acid.

3-2. Inhibition

Sonicated KATO-III antigen was absorbed in each well of a 96 wells microplate. KA3A3 antibody, whose concentration was varied, was placed in each well as an antibody inhibitor, and allowed to react at 37°C for 1 hour. After washing, the KA3A3 antibody, or NS19-9 antibody labelled with POD (the latter was a part of the reagent kit "Imunoclone CA19-9", of Fujirebio Inc.), was put into each well to react with the immobilised antigen, and the inhibition effect of the antibody inhibitor was examined in this way.

The results are shown in Figure 1. In the drawing, a circle indicates POD-labelled KA3A3, and a triangle indicates POD-labelled NS19-9 antibody. As shown in the drawings, KA3A3 antibody inhibited the reactions of the labelled KA3A3 antibody and the labelled NS19-9 antibody with the antigen.

3-3. Correlation in Serum Samples

CA19-9 antigen was measured with 62 CA19-9 positive sera, by enzyme immunoassay using POD-labelled KA3A3 monoclonal antibody POD-labelled TM19-9 monoclonal antibody, and the results obtained were correlated.

The results are shown in Figure 2. The correlation coefficient was 0.82, and there is a strong correlation between them. That is the results indicate that KA3A3 as well as TM19-9 recognises CA19-9.

3-4. Reactions with Glycolipids

Glycolipids were extracted from the carcinoma cells COLO-25 from the colon with a solvent mixture of methanol: chloroform : $H_2O$ = 2 : 1 : 0.8 (by volume), and separated into neutral glycolipid fractions and acidic glycolipid fractions by acetic acid-form DEAE-Sephadex A-25 column.

The reactivity of each fraction was measured by using POD-labelled KA3A3 monoclonal antibody material, POD-labelled NS19-9 antibody and POD-labelled anti Lewis a ($Le^a$) polyclonal antibody material. The results are shown in Figure 3. In the drawing, the full line indicates the fractions not treated with neuraminidase, and the dotted line indicates the fractions treated with neuraminidase. The circles represent the reactivity to POD-labelled KA3A3 antibody, the triangles represent the reactivity with respect to POD-labelled NS19-9 antibody, and the squares represent the reactivity with respect to POD-labelled anti $Le^a$ antibody. As a result, it can be seen that the neutral glycolipid fractions reacted strongly with anti $Le^a$ antibody, but they did not react with KA3A3 antibody. In contrast to this, the acidic glycolipid fractions reacted strongly with KA3A3 antibody, but they did not react with anti $Le^a$ antibody. However, when the acidic fractions were treated with neuraminidase, these fractions reacted with anti $Le^a$antibody but did not react with KA3A3 antibody. These facts indicate that there is a strong possibility that KA3A3 antibody is sialyl-$Le^a$. NS19-9 antibody also showed the same reaction patterns as KA3A3 antibody.

From these results, it was confirmed that the antigenic determinant recognised by KA3A3 antibody is CA19-9 (sialyl $Le^a$).

## EXAMPLE 2

### Preparation of Sensitised Particles

1. Sheep Erythrocyte carrier particles

Sheep erythrocytes were fixed with formalin in conventional manner, and suspended in 0.15 M phosphate buffer solution of pH 6.0 (0.15 M (6.0)) in a concentration of 5 v/v %. A benzoquinone 0.15 M PB (6.0) solution was added to this erythrocyte suspension, and incubated at 37°C for 30 minutes with occasional stirring. The erythrocytes were washed three times with saline solution, and suspended in 0.15 M PB (7.2) in a concentration of 5 v/v %. An equal volume of 40 µg/ml KA3A3 antibody 0.15 M PB (7.2) solution was added to the benzoquinone-treated erythrocytes suspension, and incubated at 37°C for 1 hour. The erythrocytes were washed three times with saline solution, and suspended in a dispersion medium followed by lyophilisation.

The lyophilised erythrocytes were combined with lyophilised control erythrocytes which had been sensitised in the same manner as above with an antibody to an antigen different from the object antigen to be measured, a control positive serum and a serum diluent to make a reagent kit for the detection of CA19-9.

2. Gelatin carrier particles

Gelatin particles prepared by the same method as described in Example 1 of US-A-4,416,813 were suspended in 0.15 M phosphate buffered saline solution of pH 7.2 (0.15 M PBS (7.2)) containing tannic acid in a concentration of 2.5 v/v %, and warmed at 37°C for 10 minutes. The gelatin particles were washed three times with saline solution, and suspended in 0.15 M PBS (7.2). An equal volume of 40 µg/ml KA3A3 antibody 0.15 M PB (7.2) solution was added to the tannic acid-treated gelatin particles suspension, and warmed at 37°C for 1 hour. The gelatin particles were washed three times with a saline solution, and suspended in a dispersion medium followed by lyophilisation.

3. Chicken erythrocyte particles

Chicken erythrocytes were fixed with formalin in a conventional manner, and suspended in 0.1 M PB (7.2) in a concentration of 5 v/v %. This erythrocyte suspension was mixed with toluene diisocyanate (TDI) emulsion in a final concentration of 2.5 v/v %, and stirred at 37°C for 45 minutes. The erythrocytes were washed three times with saline solution, and centrifuged to obtain precipitates of TDI-treated erythrocytes. 40 µg/ml of KA3A3 antibody 0.1 M PB (7.2) solution were added to the precipitates of TDI-treated erythrocytes whose concentration became 5 v/v %, and the mixtures were warmed at 37°C for 1 hour. The erythrocytes were washed three times with saline solution, and suspended in a dispersion medium followed by lyophilisation.

EXAMPLE 3

1. Detection of Low Titer Serum

The CA19-9 from 419 sample sera having a RIA value of less than 200 U/ml were measured by using the reverse passive particle agglutination reagent of the invention in which carrier particles were gelatin particles, and the results are shown in Table 2.

Table 2

| CA19-9 RIA (U/ml) | | | The number of Samples | The Number of Positive Sera | | Positive Sera (%) | |
|---|---|---|---|---|---|---|---|
| | | | | Final Dilution Ratio | | Final Dilution Ratio | |
| | | | | X 40 | X80 | X40 | X80 |
| | ~ | 6 | 17 | 0 | 0 | 0 | 0 |
| 7 | ~ | 36 | 127 | 33 | 8 | 26.0 | 6.3 |
| 37 | ~ | 60 | 121 | 106 | 45 | 87.6 | 37.2 |
| 61 | ~ | 100 | 107 | 104 | 45 | 97.2 | 42.1 |
| 101 | ~ | 200 | 47 | 47 | 42 | 100 | 89.4 |

2. Specificity

In order to investigate the correlation between the measured value obtained by using the reagent of the invention whose carrier particles were gelatin particles and RIA, CA19-9 of various sample sera were measured by both methods, and the results are shown in Figure 4. As shown in the drawing, a good correlation was obtained between them.

3. Comparison with EIA

Measurements with 346 sera of various cancer patients, were carried out by using the reagent of the invention whose carrier particles were gelatin particles and EIA, and the results are shown in Table 3. As shown in the table, both assays indicate a similar positive ratio.

Table 3

| Cancer | The RPPA number of Positive Sera (%) | CA19-9 EIA The number of Positive Sera (%) |
|---|---|---|
| Pancreas | 7/7 (100) | 7/7 (100) |
| Gallbladder | 24/40 (60.0) | 28/40 (70.0) |
| Bile Duct | 0/11 (0) | 0/11 (0) |
| Gastric | 14/60 (23.3) | 15/60 (25.0) |
| Esophagus | 9/20 (45.0) | 7/20 (35.0) |
| Colon,Rectum | 7/27 (25.9) | 7/27 (25.9) |
| Liver | 42/58 (72.4) | 41/58 (70.7) |
| Mammary | 2/50 (4.0) | 2/40 (4.0) |
| Lung | 17/50 (34.0) | 16/50 (32.0) |
| Others | 5/23 (21.7) | 6/23 (26.1) |

## Claims

1. An Igm monoclonal antibody having high affinity for an antigenic determinant of CA19-9 when used in a reverse passive particle agglutination reagent.

2. A reagent for use in reverse particle agglutination for detecting CA19-9 antigen, the reagent comprising an IgM class monoclonal antibody recognising an antigenic determinant of CA19-9 sensitised on carrier particles.

3. A reagent as claimed in claim 2, where the carrier particles are animal erythrocytes, gelatin particles, polystyrene latex particles or kaolin or carbon particles.

4. A reagent as claimed in claim 3, wherein the animal erythrocytes are chicken or sheep erythrocytes.

5. A reagent as claimed in claim 2, 3 or 4, wherein sensitisation of the antibody on the carrier particles has been carried out using tannic acid glutaraldehyde, bisdiazobenzidine, toluene diisocyanate, difluorodinitrobenzene, carbodiimide, quinone or chromium chloride as coupling agent.

6. A reagent as claimed in any one of claims 2 to 5, when forming part of a kit of materials for use in the diagnosis of cancer, in particular a cancer of the digestive tract, the kit additionally comprising a serum diluent, a control serum, unsensitised carrier particles and a non-specific reaction absorber.

7. An immunoassay method for the diagnosis of cancer, wherein an antigen-containing body fluid is subjected to reverse particle agglutination using a reagent for detecting CA19-9 antigen as claimed in any one of claims 2 to 5.

FIG. 1

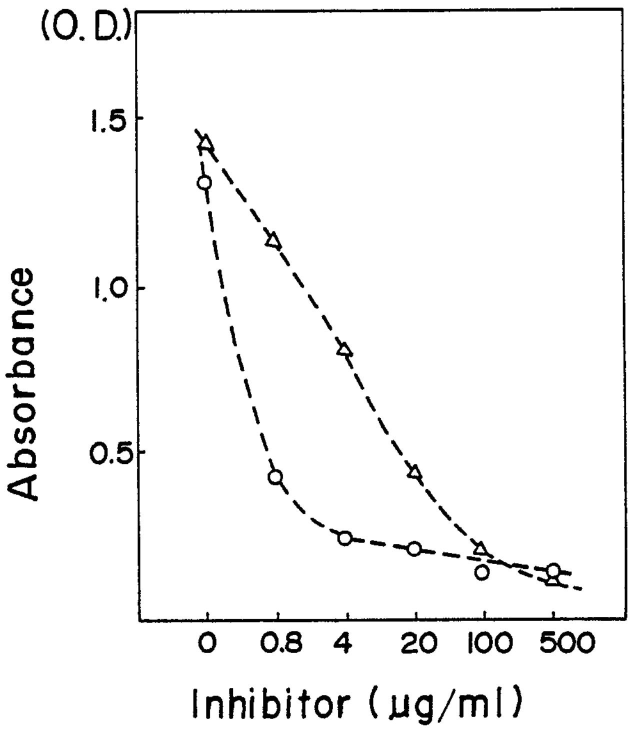

(O.D)
1.5
1.0
0.5
Absorbance
0
0.8
4
20
100
500
Inhibitor (μg/ml)


FIG. 2

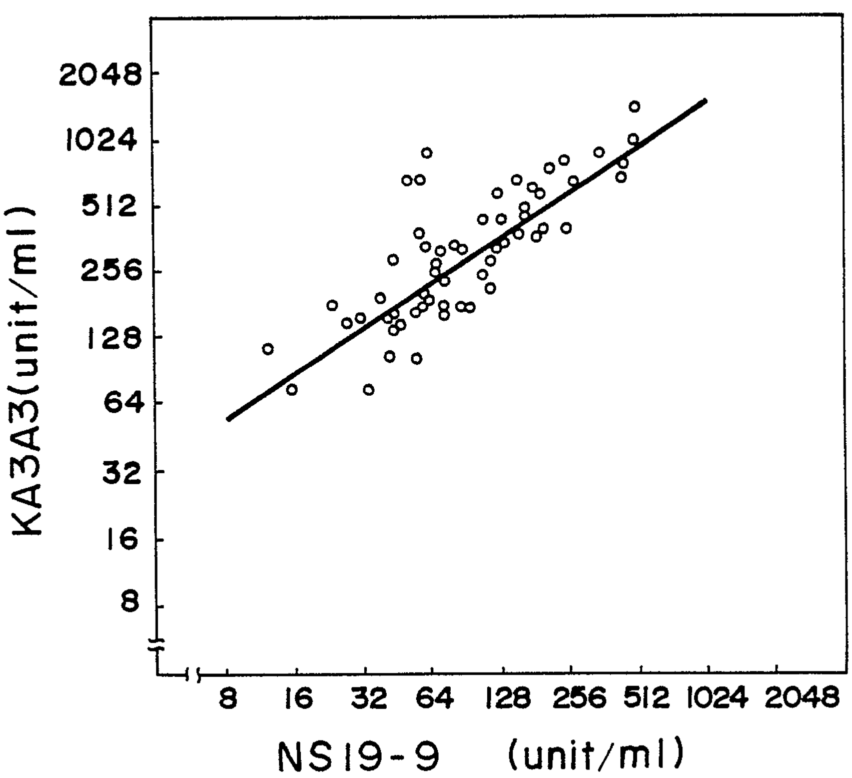

2048
1024
512
256
128
64
32
16
8
KA3A3(unit/ml)
8
16
32
64
128
256
512
1024
2048
NS19-9 (unit/ml)

FIG.3

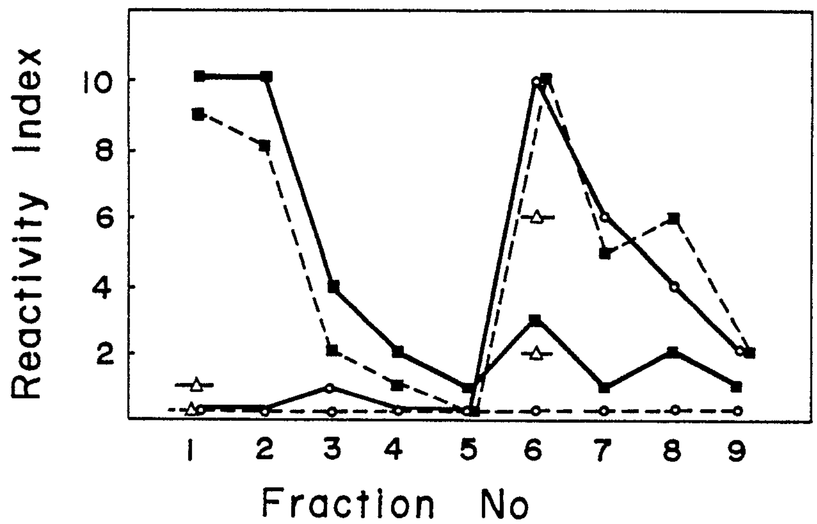
Reactivity Index
10
8
6
4
2
1 2 3 4 5 6 7 8 9
Fraction No

FIG.4

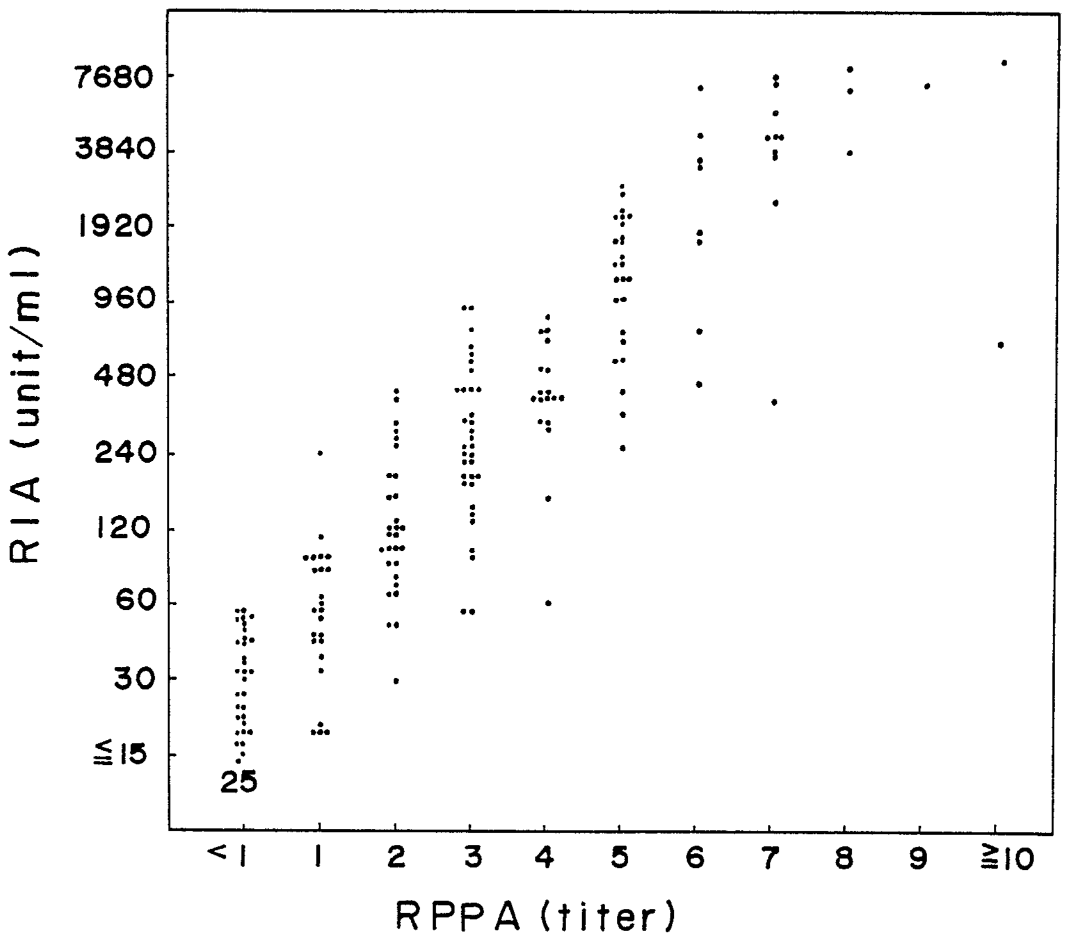
RIA (unit/ml)
7680
3840
1920
960
480
240
120
60
30
≦15
25
<1 1 2 3 4 5 6 7 8 9 ≧10
RPPA (titer)

European Patent Office

# EUROPEAN SEARCH REPORT

Application Number

EP 87 31 1262

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 165 811 (THE REGENTS OF THE UNIVERSITY OF CALIFORNIA)<br>* Page 3, line 33 - page 4, line 25; page 23, lines 1-18; page 24, table 7 * | 1-3,6,7 | G 01 N 33/577<br>G 01 N 33/574<br>G 01 N 33/543<br>G 01 N 33/555<br>C 12 P 21/00 //<br>(C 12 P 21/00<br>C 12 R 1:91 ) |
| X | EP-A-0 161 941 (NIPPON KAYAKU K.K.)<br>* Page 2, line 9 - page 3, line 21 * | 1 | |
| A | WO-A-8 400 758 (CENTOCOR INC.)<br>* Page 2, lines 20-26; page 3, lines 11-18 * | 1 | |
| A | EP-A-0 054 249 (TORAY INDUSTRIES INC.)<br>* Page 1, line 1 - page 2, line 8 * | 2,3,4,7 | |
| A | GB-A-1 577 131 (WHITLEY)<br>* Page 1, line 19-23; page 2, line 73 * | 4 | |
| A | FR-A-2 219 973 (SCHERING AG)<br>* Page 2, lines 30-37 * | 5 | TECHNICAL FIELDS SEARCHED (Int. Cl.4) |
| A | WO-A-8 201 072 (MASSACHUSETTS GENERAL HOSPITAL)<br>* Claims 10,15,16,18-20 * & EP-A-59754 ( Cat. D ) | 1-7 | G 01 N<br>C 12 P |

The present search report has been drawn up for all claims

| Place of search | Date of completion of the search | Examiner |
|---|---|---|
| THE HAGUE | 16-03-1988 | CARTAGENA Y ABELLA P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document